# EUROPEAN PATENT APPLICATION

(11) **EP 4 424 649 A1**
(43) Date of publication of application: **04.09.2024**
(21) Application number: 24160391.9
(22) Date of filing: 28.02.2024
(51) Int. Cl.: C02F 9/00, B01D 53/18, B01D 53/50, B01D 53/62, B01D 53/78, C02F 1/56, C02F 1/66, C02F 11/04, C02F 11/12, C02F 101/16

(54) **METHOD AND PLANT FOR TREATING BIOGAS**

(30) Priority: 28.02.2023 IT 202300003570
(71) Applicant: SDG S.r.l., 36015 Schio (VI) (IT)
(72) Inventor: MARTINI, Giambattista, 36015 Schio (VI) (IT)
(74) Representative: Gallo, Luca

(57) **Abstract**

Method for treating biogas, comprising a step of providing biogas (Vg), comprising a methane fraction (V_{CH4}) and at least one contaminant compound (C), and waste water (Vr) to be treated, comprising at least one organic pollutant compound (I).

In addition the method comprises a step of adding an alkaline reagent (Ra) in the waste water (Vr) in order to obtain alkaline waste water (Va) in which the organic pollutant compound (I) is transformed into modified molecules (M) due to the action of the alkaline reagent (Ra).

In addition, the method comprises a step of coagulating the modified molecules (M) in the alkaline waste water (Va) in order to obtain mature waste water (Vm) containing at least one flocculation (F) of the modified molecules (M).

In addition, the method comprises a step of purifying the biogas (Vg), in which the biogas (Vg) is placed in contact with at least one fraction of the mature waste water (Vmx) or one derivative thereof in order to release the contaminant compound (C) into the fraction of mature waste water (Vmx) or derivative.

## Description

### Field of application

The present invention regards a method and a plant for treating biogas according to the preamble of the respective independent claims 1 and 6.

The present method and plant for treating biogas are advantageously intended to be used in order to remove, from a biogas, contaminant compounds contained therein and derivatives from the process of production of the biogas itself and advantageously for enriching the methane content. For example, the present method and plant can be adapted to treat biogas generated by anaerobic digestion in dumps that receive high volumes of biomass, in livestock farms where the manure produced by the animals is transformed, or in treatment plants for sludge derived from waste water.

The present method and plant are therefore inserted in the field of treatment and purification of biogas, preferably obtained from waste water treatment plants.

### State of the art

In the field of production of biogas, plants are known comprising an anaerobic digester, which at its interior contains a biomass to be treated and one or more bacterial cultures adapted to produce a volume of biogas, by means of the anaerobic digestion of the biomass, and a digestate. In particular, the anaerobic digestion is a process of biological type, which occurs in the absence of oxygen (anaerobiosis) by means of biochemical reactions by specific bacteria present in the aforesaid bacterial cultures.

The biogas production plants are generally integrated livestock farms in order to use the manure produced by the animals as biomass, or in waste water treatment plants in order to use the sludge derivatives from the treatment of the latter as biomass.

In addition, the biogas can also be generated in a substantially spontaneous manner in dumps by the degradation of the organic component of the waste. In such case, if it is not suitably controlled and extracted by means of a recovery and waste-to-energy plant, the biogas is released into the atmosphere.

In general, such biogas consists of a mixture of gases, produced during the process of anaerobic digestion, mainly containing methane (comprised between about 50 and 75% by volume) and carbon dioxide (comprised between about 25 and 45% by volume) and is generally intended to produce thermal energy, or together produce electrical and thermal energy, for example by means of its combustion in a cogenerator or in a combustion motor.

Biogas possesses, in particular, a heat power that is a function of the methane content (on average comprised between 20000 and 24000 kJ/Nm) and in any case lower, for example, than the heat power of natural gas, since the latter contains a greater percentage of methane (about 90%) and of other hydrocarbons, such as for example ethane.

The biogas can then be subjected to an enrichment process in order to obtain biomethane, increasing the percentage of methane contained therein and consequently increasing the quality and heat power and bringing the latter to values comparable with natural gas.

In addition, it is known that the biogas produced within the anaerobic digester generally contains one or more contaminant compounds which can obstruct the combustion of the methane present in the biogas, decreasing the heat power thereof, or, following the combustion of the latter, they remain within the combustion gases and are freed into the atmosphere. For example, such contaminant compounds can be hydrogen sulfide, water (in vapor or gas form, i.e. moisture, generally comprised between 2 and 7% by volume of the biogas), volatile organic compounds (VOC, in particular hydrocarbons, oxygenated and halogenated hydrocarbons), silicon organic compounds (siloxanes, etc.), oxygen, nitrogen, ammonia, dirt, oils, solid particulate, etc. Such contaminant compounds mainly depend on the substrate, i.e. on the biomass, used for the anaerobic digestion.

Therefore, over the years, different plants and methods have been developed for cleaning the biogas exiting from the anaerobic digester, in a manner such to remove undesired components, i.e. the carbon dioxide and the contaminant compounds, both for environmental purposes and for use of the final product, and in a manner such to improve the thermal characteristics of the gas, increasing the values of the heat power.

For example, methods are known for treating biogas in order to remove the contaminant compounds, which initially provide for a step of dehydration of the biogas in order to remove at least one part of the moisture, a subsequent step of desulfurization in order to remove hydrogen sulfide and a step of removal of other undesired components.

In addition, the treatment can provide for, before or after the aforesaid steps, an enrichment step, in the jargon termed "upgrading", in order to remove at least part of the carbon dioxide present in the biogas.

In order to execute such treatment of the biogas, different plants are therefore known which comprise units of different type, each arranged for removing a fraction of the carbon dioxide and/or the contaminant compounds in the biogas.

For example, in order to carry out the removal of the carbon dioxide in the upgrading treatment, units are known comprising one or more washing columns, which are arranged for washing the biogas by means of spraying, in counter-current, of a solvent such as water (in such case the process is termed water scrubbing in the jargon) or an organic solvent, such as ethylene glycol. In this case such washing columns exploit an absorption mechanism which is based on the passage into the solvent of the carbon dioxide due to the greater solubility of the latter with respect to the methane in the aforesaid solvent.

In addition, sometimes an amine is used as organic solvent, e.g. monoethanolamine (MEA), methyldiethanolamine (MDEA) or diethanolamine (DEA). In this case such washing columns exploit an absorption mechanism of chemical type, in which the carbon dioxide is also involved in a chemical reaction with the solvent.

The solvent used can then be regenerated (an operation which is necessary in the case of organic solvents). In the event in which the solvent is water, the regeneration generally occurs by means of stripping of the carbon dioxide from the solvent with air. Otherwise, in the event in which the solvent is an organic solvent, the regeneration occurs by means of heating the solvent and/or application of vacuum. Such unit with washing columns is generally also used for the removal of contaminant compounds such as hydrogen sulfide, which also has greater solubility in the abovementioned solvents. Nevertheless, if the solvent is subsequently regenerated, hydrogen sulfide must be removed from the biogas with a different method, previously with respect to the carbon dioxide, since otherwise it would generate problems of corrosion during regeneration.

Units are also known for carrying out the removal of the carbon dioxide, which exploit other processes of physical type, for example providing for the adsorption under pressure, oscillating on zeolite substrates, molecular sieves of carbon or active carbons.

Such units are generally also used for the removal of contaminant compounds such as oxygen and nitrogen. In addition, units comprising active carbons are used in order to also remove the siloxanes and the halogenated hydrocarbons. In such case, nevertheless, other contaminant compounds such as water and hydrogen sulfide must be previously removed, since they contaminate the substrate.

Other units which exploit physical principles comprise polymer or inorganic filtering membranes, e.g. made of alumina, carbon or zeolite. Also in this case, contaminant compounds like water and hydrogen sulfide generally must be removed ahead of time, since they contaminate the membranes.

With regard to other methods for the removal of contaminant compounds, in order to remove hydrogen sulfide, as an alternative to the above-described units, units are known comprising active carbons impregnated with potassium iodide or sulfuric acid, which are adapted to catalytically convert the H₂S to elementary sulfur and water upstream of the enriching step. The carbon containing sulfur can be regenerated or substituted when it is saturated.

In addition, units are known comprising a base material, for example steel wool, wood or pellets of red sludge from the production of the aluminum, impregnated with hydroxide or iron oxide, which are adapted to react with hydrogen sulfide in order to form iron sulfide. The impregnated base material, also in this case, can be regenerated or changed.

In order to remove the water vapor (the ammonia generally together therewith), units are known which can exploit the condensation of the water by means of refrigeration, the absorption by means of glycols or hydroscopic salts, or the adsorption on the surface of a drying agent, such as silica gel or aluminum oxide. The plants for treating biogas of known type described up to now have in practice shown that they do not lack drawbacks.

A first drawback lies in the fact that the aforesaid plants for treating biogas have considerable size and are very costly, since they comprise different treatment units for each component, to be removed with a different method.

A further drawback lies in the fact that the aforesaid plants for treating biogas are costly from the energy standpoint since they employ high operating pressures, in particular the pressures of the fluids for washing the biogas, or high temperatures, for example for heating during regeneration of the adsorbing substrates.

A further drawback lies in the fact that the aforesaid plants require great quantities of chemical reagents, since several of the latter are not easily regeneratable or a fraction thereof is lost, even in the case in which they are regenerated.

A further drawback lies in the fact that the abovementioned drawbacks ensure that the plants of known type also have considerable environmental impact.

### Presentation of the invention

In this situation, the problem underlying the present invention is therefore that of overcoming the drawbacks manifested by the abovementioned solutions of known type, by providing a method and a plant for treating biogas which are capable of ensuring a high energy savings with respect to the conventional biogas treatments of known type.

A further object of the present invention is to provide a plant for treating biogas, which does not require large sizes.

A further object of the present invention is to provide a method and a plant for treating biogas, which require lower consumption of chemical reagents.

A further object of the present invention is to provide a method and a plant for treating biogas, which allow decreasing the environmental impact.

A further object of the present invention is to provide a method and a plant for treating biogas, which are less costly to attain.

### Brief description of the drawings

The technical characteristics of the invention, according to the aforesaid objects, can be clearly found in the contents of the below-reported claims and the advantages thereof will be more evident in the following detailed description, made with reference to the enclosed drawings, which represent a merely exemplifying and non-limiting embodiment of the invention, in which:
- Figure 1 shows a schematic representation of the method for purifying waste water according to a preferred embodiment of the present invention;
- Figure 2 shows a schematic representation of the plant for purifying waste water according to a preferred embodiment of the present invention.

### Detailed description of a preferred embodiment

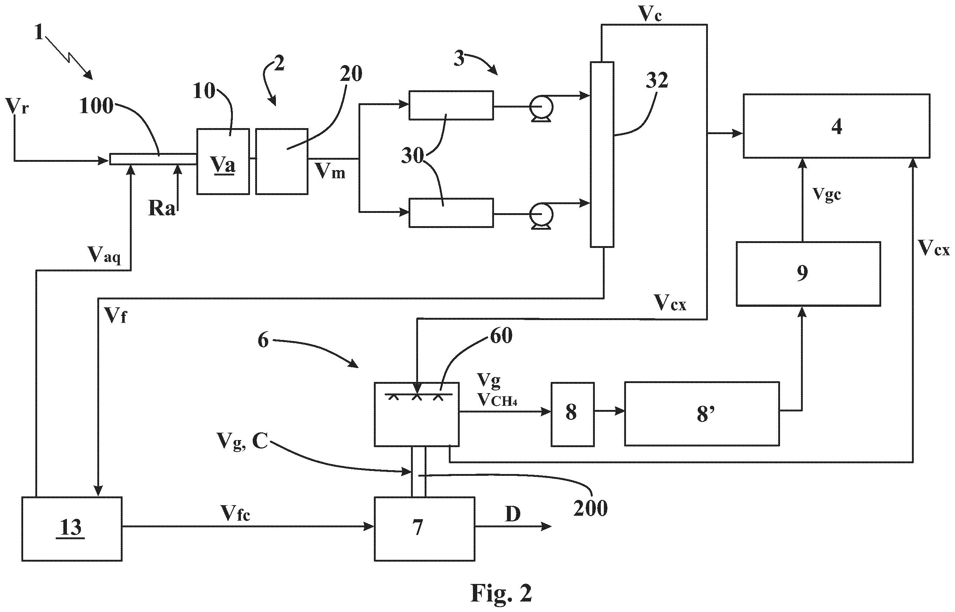

With reference to the enclosed drawings, in particular to figure 2, reference number 1 indicates the plant for treating biogas, object of the present invention, which is advantageously intended to operate according to a method for treating biogas described hereinbelow.

In particular, the biogas Vg to be treated comprises at least methane V_{CH4}, a contaminant compound C and optionally carbon dioxide, and is advantageously obtained by means of anaerobic digestion in an anaerobic digester. More in detail, the anaerobic digestion provides, in a known manner, that one or more bacterial cultures execute a series of biochemical reactions on a biomass (or substrate) in the absence of oxygen, obtaining as product a volume of the aforesaid biogas Vg and a digestate.

Advantageously, the plant 1 is advantageously intended to be used for removing, from a biogas, contaminant compounds contained therein and advantageously in order to remove at least part of the carbon dioxide and for the enrichment of methane contained in the biogas. As a non-limiting example, the plant can be arranged for treating biogas generated by anaerobic digestion in dumps, in livestock farms, or in waste water treatment plants. Preferably, the plant for treating biogas 1 is integrated in or combined with a waste water treatment plant, advantageously in order to treat the biogas generated by the plant itself, as will be described more in detail hereinbelow.

The plant 1 for treating biogas, object of the present invention, comprises a first flow piping 100 arranged for receiving and conveying waste water Vr to be treated, and such waste water Vr comprises at least one organic pollutant compound I. Advantageously, the organic pollutant compound I comprises at least one from among: a carbohydrate, a protein, a lipid, a surfactant, a dye, a biological material.

Hereinbelow with the term waste water, an aqueous suspension will be intended with suspended solid content lower than 50000 mg/l, preferably lower than 20000 mg/l. Above such concentration, it is in fact particularly difficult, requiring a lot of energy, to move the waste water.

In particular, if the plant 1 receives city waste water, by first flow piping 100 it can be intended the terminal portion of the sewage network that conveys the waste water, termed "black waters" in jargon, to the purifier, which in such case is substituted by the plant 1, object of the invention.

The plant 1 can advantageously operate according to an intermittent process (i.e. a process of batch type, with pre-established volumes of waste water, discontinuously treated) and/or according to a continuous process (i.e. with a continuous current of entering waste water) and/or according to a mixed process. Preferably, the plant 1 operates according to a continuous process.

Advantageously, the plant 1 is provided with a pretreatment unit, which is placed upstream of the first flow piping 100 and comprises at least one device selected from among: a filtration grid, desander, a grease trap.

Advantageously, the plant 1 also comprises a first tank 10, placed downstream of the first flow piping 100, in order to receive and store the waste water Vr to be treated.

Such first tank 10 is advantageously employed as a storage tank in the event in which the plant 1 provides for treating the waste water Vr continuously, allowing providing for possible interruptions of the running of waste water Vr from the first flow piping 100 towards the first tank 10 for a time equal to the ratio between the volume of the first tank 10 and the flow of waste water Vr exiting therefrom.

In addition, the plant 1 comprises metering means configured for metering an alkaline reagent Ra in the waste water, coming from the first flow piping 100 and obtaining alkaline waste water Va.

Preferably, as will be better illustrated in the method described hereinbelow, the alkaline reagent Ra is a strong base selected from among sodium hydroxide, potassium hydroxide, lithium hydroxide and their mixtures or compounds. More preferably the alkaline reagent Ra is selected from among sodium hydroxide, potassium hydroxide and their mixtures or compounds. Still more preferably, the alkaline reagent Ra is sodium hydroxide.

In particular, the metering means for metering the alkaline reagent Ra are placed at the first flow piping 100 and/or at the first tank 10.

In particular, the metering means are susceptible of adding, in multiple introduction points, the alkaline reagent Ra along the first flow piping 100 in order to allow a more effective mixing of the same alkaline reagent Ra within the waste water Vr in the first flow piping 100.

As described more in detail hereinbelow, the alkaline reagent Ra is intended to react with the organic pollutant compound I in order to hydrolyze the molecules of the latter and transform them into modified molecules M, in particular hydrolyzed or denatured.

In addition, the plant 1 is provided with a maturation unit 2 in fluid communication with the first flow piping 100, and in which alkaline waste water Va coming from the first flow piping 100 (or advantageously from the first tank 10) is susceptible of being introduced in order to obtain mature waste water Vm.

In particular, the maturation unit 2 comprises at least one second tank 20 in fluid communication with the first flow piping 100 and preferably with the first tank 10.

Advantageously the plant 1 comprises means for transferring the alkaline waste water Va along the first flow piping 100 (or from the first tank 10) to the second tank 20, such as for example one or more centrifugal pumps.

In operation, the alkaline waste water Va, which advantageously starts to react within the first flow piping 100 (or in the first tank 10), is preferably retained in the second tank 20 for a maturation time tm sufficient for completing the reaction, during which the hydrolyzed molecules M are intended to coagulate in order to form a flocculation F and obtain mature waste water Vm.

Advantageously, the plant 1 comprises further metering means in order to meter a polyelectrolyte P into the second tank 20 or into the first flow piping 100 in order to accelerate the formation of the flocculation F.

In accordance with the preferred embodiment of the invention, the plant 1 comprises a separation unit 3 in fluid communication with the maturation unit 2. In particular, the separation unit 3 is arranged for separating the mature waste water Vm coming from the maturation unit 2 into at least clarified waters Vc and sludge Vf.

Advantageously, the separation unit 3 comprises a clarifier 30 and/or separation means 32, which are preferably selected from among: a separation column, a tangential filter, an ultrafiltering membrane, a centrifuge. These apparatuses are known to the man skilled in the art and therefore will not be described in detail hereinbelow. Preferably, the separation unit 3 comprises both the clarifier 30 and the separation means 32.

Of course, without departing from the protective scope of the invention, the maturation unit 2 and the separation unit 3 can be attained as a single unit. For example, the alkaline waste water Va can carry out or complete the maturation while within the separation unit 3 in order to sediment.

In addition, the plant 1 is provided with a second flow piping 200, arranged for receiving and conveying biogas Vg to be treated, and such biogas Vg comprises at least methane V_{CH4} and a contaminant compound C, as anticipated above.

In particular, the contaminant compound C is advantageously present in molecular form in the biogas and is substantially generated during the production of the biogas itself.

For example, the contaminant compound C is a compound selected from among: water, hydrogen sulfide, ammonia, silicon organic compounds, volatile organic compounds, oxygen, nitrogen, dirt, oils, solid particulate.

More in detail, the water is advantageously present in vapor or gas form, i.e. in the form of moisture.

Advantageously, in addition, hydrogen sulfide (or hydrogen sulfide, H₂S) is formed in the course of the anaerobic digestion and its formation is tied to the equilibrium of the sulfides (S²⁻). In particular, such compound is generated by the reaction of the sulfides with water according to the equilibrium reactions (1) and (2) reported hereinbelow, dependent on the pH:

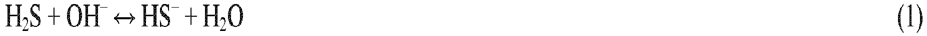

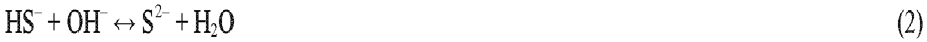

Advantageously, in addition, the content of hydrogen sulfide is a function of the concentration of the undissociated acid present in the digestate and of the Henry's law constant at the reference temperature. The sulfides are generated in turn by the decomposition of the proteins or, in the presence of sulfates, by the action of sulfate-reducer bacteria, which oxidize the organic substance, reducing the sulfur to sulfide. Not all the sulfides react with the water to generate hydrogen sulfide, since a part can react with metals such as copper, lead, zinc, mercury and generate metal sulfides, which precipitate as insoluble minerals. Advantageously, in addition, the ammonia is formed during the anaerobic degradation of protein-rich substrates. In particular, the concentration of ammonia in the biogas is very low, for example lower than 100 ppm in the biogas from an anaerobic digester and about 5 ppm in the dump biogas. Advantageously, the concentration of ammonia is a function of pH, temperature and ratio between carbon and nitrogen (C/N).

More in detail, a high pH value, common in a thermophilic digestion process, leads to a greater concentration of ammonia in the biomass for this process type. Otherwise, the quick mixing of the biomass contributes to the increase of the concentration of ammonia in the biogas, since it facilitates the transfer from the liquid phase to the gas phase.

Advantageously, in addition, the silicon organic compounds, such as siloxanes, are compounds of organosilicon synthesis, which in particular contain a linear or branched chain of Si-O₂ atoms, in which organic radicals (e.g. methyl, ethyl, etc.) can be bonded to the silicon atom. For example, the simplest siloxanes with linear chain can be hexa-methyl-di-siloxane and octo-methyl-cyclotetra-siloxane. In the biogas, volatile siloxanes with low molecular weight are in particular present.

More in detail, the silicon organic compounds are present in products of common use such as cosmetics and drugs. Therefore, such contaminant compounds C are advantageously situated above all in the dump biogases and in those deriving from digestion of the sludge from waste water treatment. Such compounds in particular are damaging if present in the biogas, since during the combustion of the latter they are converted into inorganic silicon, determining corrosive phenomena in the components of the burner or motor.

Advantageously, in addition, the volatile organic compounds present in the biogas comprise hydrocarbons, such as for example alkanes, aromatic hydrocarbons, cycloalkane, terpenes; oxygenated hydrocarbons, such as for example alcohols and ketones; and/or halogenated hydrocarbons. In particular, the volatile organic compounds present in the biogas coming from the dump are mainly aromatic hydrocarbons, such as ethylbenzene, xylenes and/or toluene; aliphatic hydrocarbons, such as for example nonane; and terpenes, such as for example α-pinene. Advantageously, the total content of volatile organic compounds in the biogas coming from dumps is comprised between 46 and 173 mg/m³. Advantageously, in addition, the total content of volatile organic compounds in the biogas coming from anaerobic digestors is comprised between 13 and 268 mg/m³.

Advantageously, the second flow piping 200 is made of AISI 316 steel, of plastic material or of any other material capable of resisting corrosion, from possible corrosive compounds present in the biogas Vg to be treated.

In accordance with the idea underlying the present invention, the plant 1 comprises a purification unit 6, in fluid communication with the second flow piping 200 and at least the maturation unit 2. In addition, the purification unit 6 is arranged for receiving the biogas Vg and at least one fraction of the mature waste water Vmx or one derivative thereof and for placing them in contact with each other in order to transfer the contaminant compound C from the biogas Vg to the fraction of mature waste water Vmx or derivative.

Preferably, the derivative of the mature waste water Vm comprises the clarified waters Vc, obtained through the separation unit 3 described above, and preferably is constituted thereby.

Therefore, preferably, the purification unit 6 is in fluid communication with the separation unit 3 in order to receive at least one fraction of the clarified waters Vcx. In such case, the purification unit 6 is to be considered in fluid communication with the maturation unit 2 indirectly through the separation unit 3. Advantageously, therefore, the purification unit 6 is arranged for placing in contact the biogas Vg with at least one fraction of the clarified waters Vcx in order to transfer in the latter the contaminant compound C present in the biogas Vg itself.

In particular, the fraction of mature waste water Vmx or derivative thereof, i.e. preferably the fraction of the clarified waters Vex, advantageously a part of alkaline reagent Ra that has not reacted with the organic pollutant compound I. Therefore, the biogas Vg is advantageously purified, the contaminant compound C removed by means of chemical absorption, i.e. by means of at least one chemical reaction between the contaminant compound C and the alkaline reagent Ra.

For example, in the event in which the alkaline reagent Ra employed is caustic soda and the contaminant compound C to be removed is hydrogen sulfide, the latter will react with the alkaline reagent Ra according to the reaction (3) reported hereinbelow:

2 NaOH + H₂S - Na₂S + 2 H₂O (3)

In addition, the caustic soda advantageously also reacts with the carbon dioxide present in the biogas Vg according to the reaction (4) reported hereinbelow:

2 NaOH + CO₂ → Na₂CO₃ + H₂O (4)

In addition, the fraction of mature waste water Vmx or derivative thereof, i.e. preferably the fraction of the clarified waters Vex, advantageously reacts with other gaseous substances of acidic nature, such as for example SO₂ and mercaptans, neutralizing them.

Advantageously, in addition, in the fraction of mature waste water Vmx or derivative thereof, i.e. preferably in the fraction of the clarified waters Vcx, contaminated compounds C such as ammonia, siloxanes and volatile organic compounds are transferred.

In this manner, one advantageously obtains a plant capable of treating the biogas, by employing an alkaline product (the mature waste water or the clarified waters), which is a discard product and does not require being regenerated. Indeed, the waste water is intended for subsequent processing so to be able to be discharged or reused.

In addition, the plant 1, by exploiting part of a treatment of waste water in order to purify the biogas Vg, advantageously employs less chemical reagents, since it uses the alkaline reagent Ra not reacted in the treatment of the waste water.

In accordance with the preferred embodiment of the invention, the plant 1 comprises a thickening unit 13 in fluid communication with the separation unit 3. In particular, the thickening unit 13 is arranged for separating the sludge Vf coming from the separation unit 3 into an alkaline recovery liquid Vaq and concentrated sludge Vfc.

In particular, the recovery liquid Vaq comprises water and at least one fraction of alkaline reagent Ra that is unreacted in the maturation section 2.

Preferably, the thickening unit 13 is in fluid communication with the first flow piping 100 in order to reintroduce the recovery liquid Vaq in the waste water Vr to be treated. In particular, the thickening unit 13 comprises a pump arranged for moving the recovery liquid Vaq towards the first flow piping 100. In this manner, the recovery liquid Vaq is introduced into the waste water Vr and recycled, in a manner such to save on the quantity of alkaline reagent Ra introduced into the waste water Vr.

As an alternative or in combination with that described above, in accordance with one embodiment not illustrated in the enclosed figures, the derivative of the mature waste water Vm comprises the sludge Vf (or the concentrated sludge Vfc), obtained through the separation unit 3 described above, and preferably is constituted thereby.

Therefore, advantageously, the purification unit 6 is in fluid communication with the separation unit 3 (or with the thickening unit 13) in order to receive at least one fraction of the sludge Vfx (or a fraction of the concentrated sludge Vfcx). Also in such case, the purification unit 6 is to be considered in fluid communication with the maturation unit 2 indirectly through the separation unit 3 (and possibly the thickening unit 13).

Advantageously, therefore, the purification unit 6 is arranged for placing the biogas Vg in contact with at least one fraction of the sludge Vfx (or a fraction of the concentrated sludge Vfcx) in order to transfer, into the latter, the contaminant compound C present in the biogas Vg itself, in particular in a manner analogous to that already described above.

Advantageously, the plant 1 is provided with at least one anaerobic digester 7 placed downstream of the separation unit 3, and preferably also downstream of the thickening unit 13, and arranged for treating the sludge Vf or their derivatives coming from the separation unit 3 in order to generate biogas Vg and a digestate D. In particular, the biogas Vg comprises a methane fraction V_{CH4} and at least one contaminant compound C. In addition, the biogas Vg advantageously comprises carbon dioxide. Advantageously, at least part of the carbon dioxide produced in the anaerobic digester 7 and present in the biogas Vg reacts with the sludge Vf or the derivative thereof and lowers the pH thereof during the anaerobic digestion. Preferably, the derivative of the sludge Vf comprises or is constituted by the concentrated sludge Vfc coming from the thickening unit 13, or from the fraction of the sludge Vfx or from the fraction of the concentrated sludge Vfcx exiting from the purification unit 6.

Advantageously, the second flow piping 200 is placed to connect between the anaerobic digester 7 and the purification unit 6 in order to bring the biogas Vg to the purification unit 6.

In such case, the biogas Vg treated in the plant 1 derives directly from the sludge Vf obtained from the treatment of the waste water Vr.

Of course, without departing from the protective scope of the present invention, the plant 1 can advantageously be connected with the second flow piping 200 to other plants capable of generating a volume of biogas Vg for producing energy, e.g. to a dump in which high quantities of biomass are preserved, or other plants and/or farms that produce biomass.

In accordance with a first embodiment of the present invention, the purification unit 6 comprises at least one tank, which is arranged for containing the fraction of mature waste water Vmx or derivative (preferably the fraction of clarified waters Vcx).

In particular, the tank is closed and is filled preferably with mature waste water Vm or derivative (preferably the clarified waters Vc) only partially, while the remaining volume above the free surface of the liquid is left free in order to receive the purified biogas Vg.

In addition, the purification unit 6 advantageously comprises at least one bubbling dispenser placed within the tank and in fluid communication with the second flow piping 200 in order to convey the biogas Vg into the tank by means of bubbling.

Advantageously, the bubbling dispenser is placed on the bottom of the tank and is provided with a plurality of microporous diffusers, preferably made of plastic material (e.g. HPDE) or of ceramic material.

Advantageously, the biogas Vg in the second flow piping 200 is maintained at a minimum pressure comprised between about 100 and 400 mmHg, such to introduce it under pressure within the closed tank. Such over-pressure is advantageously directly generated within the anaerobic digester 7 or, alternatively, by a compressor placed to intercept the second flow piping 200.

In operation, the biogas Vg is made to flow into the tank through the micropores of the diffusers, forming bubbles of micrometric diameter.

In this manner it is possible to maximize the contact surface of the biogas Vg with the fraction of alkaline clarified waters Vcx. In addition, the microbubbles generated advantageously have a low thrust force for re-ascending along the tank, therefore also the contact time between the biogas Vg and the fraction of clarified waters Vcx is maximized.

During such contact time, the biogas Vg advantageously releases the carbon dioxide and the at least one contaminant compound C, while the methane, being highly insoluble in water, re-emerges above the free surface of the liquid in the tank, and is collected in the free volume of the tank.

Advantageously, the purification unit 6 comprises washing means, not depicted in the enclosed figures, preferably placed within the tank, and intended to remove further undesired contaminant compounds I, e.g. siloxanes.

In accordance with a second embodiment of the present invention, the purification unit 6 comprises at least one washing column 60, which comprises a treatment tank and a spraying head, placed within the treatment tank and placed in fluid communication with the separation unit 3 in order to nebulize, within the treatment tank, said the fraction of mature waste water Vmx or derivative (preferably the fraction of clarified waters Vcx).

In addition, the washing column 60 according to such embodiment comprises an introduction mouth, placed within the treatment tank below the spraying head and placed in fluid communication with the second flow piping 200 in order to introduce the biogas Vg in the treatment tank

In operation, the washing column 60 is adapted to purify the biogas Vg by means of spraying of the fraction of clarified waters Vcx in counter-current in order to remove the contaminant compound C from the biogas Vg.

In addition, the washing column 60 advantageously comprises a plurality of packing bodies placed within said treatment tank between the introduction mouth and the spraying head. In particular, the packing bodies have high specific surface area so as to increase the area of contact between the biogas Vg and the fraction of clarified waters Vex.

Advantageously, the washing column 60 is of static type, of the type known in the field and termed in the jargon "static scrubber", in which the packing bodies are fixed within the column. Alternatively, the washing column 60 is of dynamic type, of the type known in the field and termed "floating scrubber" in the jargon, in which the packing bodies are continuously moved by the biogas Vg flow that ascends along the aforesaid column. The floating scrubbers are advantageously employed for biogas Vg comprising dust to be removed.

In operation, the biogas Vg is introduced from the bottom, and ascends the column with packing bodies in counter-current, coming into contact with the fraction of clarified waters Vex and transferring to the latter carbon dioxide and the at least one contaminant compound C.

Advantageously, the plant 1 comprises a dehumidification unit 8, placed downstream of the purification unit 6 and arranged for receiving the biogas Vg and removing most of the moisture from the latter, in a manner such to ensure an optimal combustion of the methane.

In addition, the plant 1 advantageously comprises an adsorption unit 8' on active carbons, which is arranged for absorbing a further fraction of carbon dioxide from biogas Vg, so as to enrich it with methane and increase the heat power of the latter.

In this manner one obtains a plant for treating biogas which overall has low energy impact since the use of high pressures of the operating fluids is not required, in particular the fluids employed for purifying the biogas Vg.

Advantageously, in addition, the plant 1 comprises a cogenerator 9 arranged for generating at least electrical energy and a combustion gas Vgc comprising at least one fraction of carbon dioxide and one fraction of water vapor starting from the purified biogas Vg. The cogenerator 9 is advantageously hydraulically connected to the purification unit 6, to the dehumidification unit 8 or to the adsorption unit 8' in order to receive, from at least one of these, the biogas Vg. In this manner one obtains a plant 1 with low environmental impact, since the electrical energy generated due to the combustion of the biogas Vg is in part employable for serving the equipment of the plant 1 itself.

In accordance with the preferred embodiment of the plant 1 according to the invention, the fraction of clarified waters Vcx is hereinbelow connected from the bottom of the tank or of the washing column 60 is joined with the remaining clarified waters Vc in order to be subsequently treated.

For such purpose, the plant 1 is advantageously provided with a neutralization unit 4, which is in fluid communication with the separation unit 3 in order to receive from the latter the clarified waters Vc and with the purification unit 6 in order to receive from the latter the fraction of clarified waters Vcx and reintroduce it into the clarified waters Vc. In addition, the neutralization unit 4 is advantageously in fluid communication with the cogenerator 9 in order to receive from the latter the combustion gas Vgc comprising the fraction of carbon dioxide and place it in contact with the clarified waters Vc in order to obtain neutral clarified waters Vcn.

In particular, in the aforesaid neutralization unit 4, the combustion gas Vgc is preferably introduced in the clarified waters Vc via bubbling. In this manner, the carbon dioxide present in the combustion gas Vgc reacts with the unreacted alkaline reagent Ra that is dissolved in the clarified waters Vc, and lowers the pH of the latter, obtaining the neutral clarified waters Vcn, advantageously provided with a pH comprised between 7 and 8.5.

The plant 1 advantageously comprises further units for treating the neutral clarified waters Vcn, for example selected from among: an aerobic digester, filtration membrane, double exchange tanks with resins, in which the neutral clarified waters Vcn, containing the contaminant compounds C removed from the biogas Vg, can be further purified, lowering the concentration of the contaminant compounds C.

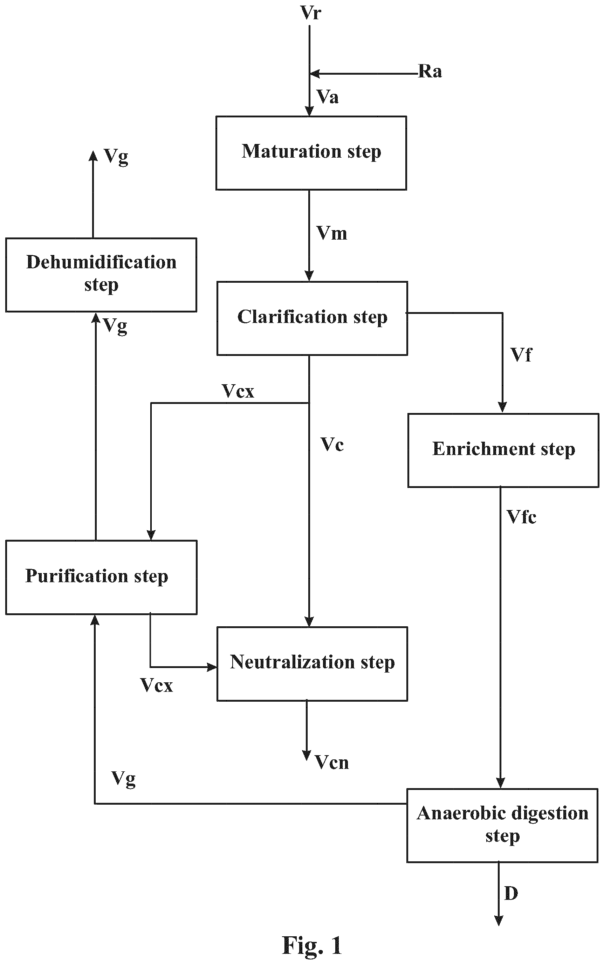

Also forming the object of the invention is a method for treating biogas, one preferred embodiment thereof being schematically illustrated in figure 1, which preferably employs the plant 1, object of the present invention, regarding which all the number references will be made for the sake of descriptive simplicity. Of course, without departing from the protective scope of the present invention, such method can employ any other plant for treating biogas which is adapted for such purpose.

According to the idea underlying the present invention, the method for treating biogas, comprises the following operating steps.

The method comprises a step of providing a biogas Vg, which comprises at least one methane fraction V_{CH4} and at least one contaminant compound C.

Advantageously, the contaminant compound C is selected from among the compounds listed above, and/or listed in Table 1 reported hereinbelow. Of course, without departing from the protection of the present invention, the contaminant compound C can also be any other compound that lowers the quality of the biogas Vg or that, if freed into the atmosphere, lowers the quality of the air, resulting polluting for the latter.

Reported hereinbelow in Table 1 are two examples of composition of biogas Vg respectively coming from the anaerobic digestion of sludge Vf coming from the treatment of waste water Vr, and from the storage of biomass in a dump.

**Table 1 - Biogas composition examples**

| | Biogas from anaerobic digestion | Biogas from dump |
|---|---|---|
| Methane [% v/v] | 60 - 70 | 35 - 65 |
| Other hydrocarbons [% v/v] | 0 | 0 |
| Hydrogen [% v/v] | 0 | 0 - 3 |
| Carbon dioxide [% v/v] | 30 - 40 | 15 - 50 |
| Nitrogen [% v/v] | ≤ 1 | 5 - 40 |
| Oxygen [% v/v] | ≤ 0.5 | ≤ 5 |
| Hydrogen sulfide [ppm vol.] | ≤ 4000 | ≤ 100 |
| Ammonia [ppm vol.] | ≤ 100 | ≤ 5 |
| Volatile organic compounds (VOC) [ppm vol.] | 10 - 300 | 40-180 |

In addition, the method comprises a step of providing waste water Vr to be treated, which comprise at least one organic pollutant compound I.

In accordance with a first example of waste water Vr, the latter is city waste water, i.e. water used in human, domestic, industrial or farming activities, which for this reason contains organic and inorganic substances. Such waste water Vr for example contain proteins, carbohydrates, cellulose, and mixtures of chemical compounds such as for example surfactants. Table 2 reports a typical characterization of the first example of waste water Vr.

**Table 2 - Typical characterization of city waste water**

| **Parameters** | **Measurement unit** | **Values** |
|---|---|---|
| pH | | 6.0 - 8.5 |
| COD | mg/l | 250 - 800 |
| Total Nitrogen (N) | mg/l | 20 - 80 |
| Ammonia Nitrogen (N) | mg/l | 15 - 60 |
| Total Phosphorous (P) | mg/l | 3 - 10 |
| Organic substance (with organic compounds such as uric acid, urea, amino acids, fatty acids, alcohols, etc.) | % of the dry matter (dm) | 31 |
| Fibrous Proteins (collagen) | % of the organic substance (os) | 34 |
| Lipids | % os | 10 |
| Carbohydrates | % os | 10 |
| Cellulose | % os | 40 |
| Mineral salts (Ca, Na, Mg, PO_{Tot.}) | % dm | 5 |
| Metals (Fe, Zn, Cu, etc.) | % dm | < 0.5 |

In accordance with a second example of waste water Vr, the latter is waste water from the textile industry and contain for example cellulose, starch, surfactants, mineral charges, inerts. The composition of such waste water Vr can vary in relation to the characteristics of the raw materials and of production processes. Table 3 reports a typical characterization of the second example of waste water Vr.

**Table 3 - Typical characterization of waste water from the textile industry**

| **Parameters** | **Measurement unit** | **Values** |
|---|---|---|
| pH | | 6.5 - 10 |
| COD | mg/l | 250 - 900 |
| Total Nitrogen (N) | mg/l | 20 - 40 |
| Ammonia Nitrogen (N) | mg/l | <15 |
| Total Phosphorous (P) | mg/l | 10 - 30 |
| Organic compounds (dyes, desizing, softeners, surfactants) | % dm | 45 |
| Inorganic compounds (soda, hypochlorite, various acids, salt) | % dm | Variable from 15 to 30 if present |
| Keratin (animal fibers) | | |
| Cellulose (plant fibers) | | |
| Synthesis fibers (acrylic, polyester, etc.) | | |
| Ash | % dm | 13 |
| Mineral salts (Ca, Na, K, Mg, PO_{Tot.}) | % dm | 12 |
| Metals (Fe, Zn, Cu, etc.) | % dm | < 0.5 |

In accordance with a third example of waste water Vr, the latter is waste water from the paper industry and contain for example cellulose, starch, surfactants, mineral charges, inerts. The composition of such waste water Vr can vary in relation to the characteristics of the raw materials and of production processes. Table 4 reports a typical characterization of the third example of waste water Vr.

**Table 4 - Typical characterization of waste water from the paper industry**

| **Parameters** | **Measurement unit** | **Values** |
|---|---|---|
| pH | | 6.2 - 8.5 |
| COD | mg/l | 1500 - 3000 |
| Total Nitrogen (N) | mg/l | 50 - 150 |
| Total Phosphorous (P) | mg/l | 15 - 25 |
| Organic substance (cellulose derivatives, charges and fillers, resins and various additives, etc.) | % dm | 15 |
| Cellulose | % dm | 60 |
| Ash | % dm | 10 |
| Mineral salts (Ca, Na, Mg, PO_{Tot.}) | % dm | 15 |
| Metals (Fe, Zn, Cu, etc.) | % dm | <0.1 |

In accordance with a fourth example of waste water Vr, the latter is waste water from the butcher industry and contains organic compounds such as for example uric acid, urea, amino acids, fatty acids, alcohols, etc. The composition of such waste water Vr varies as a function of the type and number of animals slaughtered and by the presence of complementary processing operations in the structure. Table 5 reports a typical characterization of the fourth example of waste water Vr.

**Table 5 - Typical characterization of waste water from the butcher industry**

| **Parameters** | **Measurement unit** | **Values** |
|---|---|---|
| pH | | 4.2 - 8.5 |
| COD | mg/l | 4000 - 12000 |
| Total Nitrogen (N) | mg/l | 150 - 450 |
| Ammonia Nitrogen (N) | mg/l | < 50 |
| Total Phosphorous (P) | mg/l | 25 - 50 |
| Proteins | % dm | 70 |
| Lipids | % dm | 15 |
| Carbohydrates | % dm | 10 |
| Mineral salts (Ca, Na, Mg, PO_{Tot.}) | % dm | 5 |
| Metals (Fe, Zn, Cu, etc.) | % dm | < 0.5 |

In accordance with a fifth example of waste water Vr, the latter is waste water from the dairy industry. Table 6 reports a typical characterization of the fifth example of waste water Vr.

**Table 6 - Typical characterization of waste water from the dairy industry**

| **Parameters** | **Measurement unit** | **Values** |
|---|---|---|
| pH | | 5.5 - 7.5 |
| COD | mg/l | 1250 - 3000 |
| Total Nitrogen (N) | mg/l | 50 - 200 |
| Ammonia Nitrogen (N) | mg/l | < 15 |
| Total Phosphorous (P) | mg/l | 10 - 25 |
| Proteins | % dm | 8 - 14 |
| Lipids | % dm | 17 |
| Carbohydrates | % dm | 50-70 |
| Mineral salts (Ca, Na, Mg, PO_{Tot.}) | % dm | 6 - 8 |

In accordance with a sixth example of waste water Vr, the latter is waste water from the tanning industry and for example contains surfactants, mineral charges, inerts. The composition can vary in relation to the characteristics of the raw materials and of production processes. Table 7 reports a typical characterization of the sixth example of waste water Vr.

**Table 7 - Typical characterization of waste water from the tanning industry**

| **Parameters** | **Measurement unit** | **Values** |
|---|---|---|
| pH | | 5.5 - 10.0 |
| COD | mg/l | 6000 - 12000 |
| Total Nitrogen (N) | mg/l | 200 - 400 |
| Ammonia Nitrogen (N) | mg/l | 100 - 200 |
| Total Phosphorous (P) | mg/l | 15 - 50 |
| Proteins | % dm | 42 |
| Lipids | % dm | 17 |
| Carbohydrates | % dm | 7 |
| Cellulose | % dm | 3 |
| Ash | % dm | 13 |
| Mineral salts (Ca, Na, Mg, PO_{Tot.}) | % dm | 15 |
| Metals (Fe, Zn, Cu etc.) | % dm | < 1 |

As is visible from the above-reported examples, the waste water Vr arranged in the present invention preferably has high organic load.

In particular, the organic pollutant compound I is any undesired organic molecule in the waste water Vr that must be treated in order to discharge it into the outside environment or reuse it within an industrial plant, from which waste water Vr itself derives.

For example, the organic pollutant compound I is a compound deriving from the food processing process, in particular from processing meat, fish, fruit or vegetables, or from the dairy industry, or from the paper processing process, or an organic dye deriving from the tanning industry.

Advantageously, the organic pollutant compound I comprises at least one from among: a carbohydrate, a protein, a lipid, a surfactant, a dye, a biological material.

For example, if it is a carbohydrate, the organic pollutant compound I is a polysaccharide, in particular a starch or cellulose.

In addition, if it is a dye (or a tincture), the organic pollutant compound I comprises an acid dye, an azo extension dye, a basic dye, a mordent dye, an oxygen dye, a pigment dye, a reactive dye, a substantive dye for cotton, a vat dye, or a sulfur dye. Such dyes are known in the field and therefore they will not be described more in detail.

Of course without departing from the protective scope of the present invention, the organic pollutant compound I can be free in the waste water Vr or also contained in any unicellular element (bacteria and/or virus) whose concentration in the waste water Vr to be treated is greater than the legal limits and is susceptible of being removed by means of a method for the treatment thereof. Therefore, in such case the organic pollutant compound I will more generally be considered a biological material.

In addition, the method comprises step of adding an alkaline reagent Ra in the waste water Vr in order to obtain alkaline waste water Va, in which the at least one organic pollutant compound I is transformed into modified molecules M due to the action of the alkaline reagent Ra. In particular, the modified molecules M are hydrolyzed and/or denatured molecules.

Advantageously the alkaline reagent Ra is a strong base selected from among: sodium hydroxide, potassium hydroxide, calcium hydroxide, barium hydroxide, lithium hydroxide, magnesium hydroxide, and their mixtures or compounds. In addition, the pH of the alkaline waste water Va is advantageously comprised between 10 and 14 and preferably is about 12.2.

Preferably, the alkaline reagent Ra is a strong base selected from among sodium hydroxide, potassium hydroxide, lithium hydroxide and their mixtures or compounds. More preferably the alkaline reagent Ra is selected from among sodium hydroxide, potassium hydroxide and their mixtures or compounds. Still more preferably, the alkaline reagent Ra is sodium hydroxide.

Indeed the latter monovalent alkaline metal hydroxides advantageously have high solubility within the waste water Vr and do not precipitate. In particular, once dissolved in water, such types of alkaline reagent Ra are able to cause the hydrolysis of the organic pollutant compound I without forming metal salts (e.g. carbonates, as occurs with the bivalent metal compounds, i.e. calcium, magnesium or barium, and trivalents) which would precipitate, forming an inert sludge.

In addition, such alkaline reagents Ra advantageously substantially involve only alkaline denaturing and/or alkaline hydrolysis of the organic pollutant compound I and not double exchange reactions, which would require further steps for eliminating the reaction product. Therefore, the addition of an alkaline reagent Ra, specifically a hydroxide of a monovalent alkaline metal, in order to make the waste water Vr basic, advantageously allows minimizing the steps of the method and the use of lower quantities of reagents.

In addition, the method comprises a step of coagulating the modified molecules M in order to obtain mature waste water Vm containing at least one flocculation F of the modified molecules M.

In particular, the coagulation step provides for the maturation of the alkaline waste water Va for a maturation time tm, in which the modified molecules M coagulate in order to obtain mature waste water Vm.

Advantageously, the step of maturation provides for the addition of at least one polyelectrolyte P to the alkaline waste water Va in order to accelerate the formation of the flocculation F.

Advantageously, the method comprises a separation step, preferably by means of a previously-described separation unit 3, for separating the mature waste water Vm at least into clarified waters Vc and sludge Vf. In particular, the flocculation F is mainly contained in the sludge Vf.

In addition, the method comprises a step of purifying the biogas Vg, in which the biogas Vg is placed in contact with at least one fraction of the mature waste water Vmx or one derivative thereof in order to transfer the contaminant compound C in the fraction of mature waste water Vmx or derivative.

In particular, the contaminant compound C is transferred into the fraction of mature waste water Vmx or derivative in the manner described above, i.e. by means of chemical absorption by reaction with the alkaline reagent present in the fraction of mature waste water Vmx or derivative.

More in detail, the contaminant compound C is transferred from the biogas Vg to the fraction of mature waste water Vmx or derivative through the separation surface of the two phases, i.e. their interface area. In particular, the interface area depends on the type of absorption systems employed, e.g. bubbling or washing in counter-current, as in the plant embodiments described above.

Advantageously, the flow of the fraction of mature waste water Vmx or derivative that must be used depends on the absorption system type (the liquid/gas ratio) and the minimum contact time necessary so that the absorption occurs. For example, for very soluble contaminant compounds C, low flow rates are required together with shorter contact times between the two steps, to the benefit of the investment and operating costs.

Advantageously, the transfer of the contaminant compound C is adjusted by a solubility parameter, which is advantageously dependent on the temperature of the system and on the partial pressure of the contaminant compound C. In particular, the solubility parameter increases with the decrease of the temperature and the increase of the pressure of the gaseous component.

For example, the power of being able to transfer the contaminant compound C is adjusted in accordance with the equation (5), reported hereinbelow, relative to Henry's equilibrium:

*C*x = *k·P* (5)

where Cx indicates the concentration of the contaminant compound C (previously in gas phase) dissolved in the fraction of mature waste water Vmx or derivative, P is the partial pressure of the contaminant compound C in the biogas Vg and k is Henry's law constant relative to the contaminant compound C.

The same considerations advantageously also hold true for the carbon dioxide present in the biogas Vg, which tends to be transferred from the biogas Vg to the fraction of mature waste water Vmx or derivative according to the same abovementioned principles. In particular, the low solubility of the methane, about 26 times lower than that of the carbon dioxide, ensures that the latter preferably comes into solution and reacts for example according to the reaction (4).

Advantageously, the transfer of the contaminant compound C, in particular a compound such as ammonia or hydrogen sulfide, also depends on the pH of the fraction of mature waste water Vmx or derivative. In particular, the ammonia with the lowering of the pH (e.g. due to the solubilization of the hydrogen sulfide or of the carbon dioxide) passes to ammonia ion, more easily solubilizing. In addition, since hydrogen sulfide is absorbed more rapidly than the carbon dioxide from alkaline aqueous solutions, it is possible to obtain a partial selectivity when both are present with low contact times and low temperature.

Preferably, the derivative of the mature waste water Vm comprises the clarified waters Vc, preferably it is constituted thereby. Therefore, in such case, in the purification step, the biogas Vg is placed in contact with at least one fraction of the clarified waters Vex.

In this manner, the biogas Vg is placed in contact with a solution with low solid content, since the flocculation F remains substantially entirely in the sludge Vf.

Advantageously, the fraction of clarified waters Vcx is comprised between 1% and 5% of the total of the clarified waters Vc.

Indeed, in order to purify the biogas Vg, it is necessary to have a small amount of alkaline reagent Ra, in this case contained by the fraction of clarified waters Vcx.

Of course, if the pH of the clarified waters Vc is lower than a value of about 9, a higher quantity of clarified waters Vc will be necessary, in view of the low concentration of the alkaline reagent Ra that is unreacted in the clarified waters Vc themselves.

Advantageously, moreover, the method comprises a step of thickening the sludge Vf, in which at least one alkaline recovery liquid Vaq is removed from the sludge Vf, by means of thickening, in order to obtain concentrated sludge Vfc.

In particular, the recovery liquid Vaq comprises water and at least one fraction of alkaline reagent Ra that is unreacted. Advantageously, the recovery liquid Vaq is added to the waste water Vr.

As an alternative or in combination with that described above, in accordance with an embodiment not illustrated in the enclosed figures, the derivative of the mature waste water Vm comprises the sludge Vf (or the concentrated sludge Vfc), preferably it is constituted thereby. Therefore, in such case, in the purification step, the biogas Vg is placed in contact with at least one fraction of sludge Vfx (or a fraction of the concentrated sludge Vfcx).

Advantageously, the method also comprises a step of dehumidifying the biogas Vg, following the purification step.

In this manner the biogas Vg lacks most of the moisture accumulated following the washing in the purification step, with increase of its own heat power.

Advantageously in addition, the biogas Vg, following the dehumidification step can be subjected to further treatments, for example in order to further remove the carbon dioxide and therefore increase the percentage of methane of the biogas Vg, or in order to remove contaminant compounds C which are insoluble in water or which are unable to react with the alkaline reagent Ra.

Advantageously, the method comprises a step of digesting the sludge Vf or one derivative thereof by means of an anaerobic digester 7 in order to produce biogas Vg and a digestate D.

More in detail, the digestion step provides for employing anaerobic bacteria, placed advantageously within the anaerobic digester 7 and maintained in a controlled temperature and pH environment.

In particular, the sludge Vf (or the derivative thereof) is introduced at a pH preferably comprised between 7.5 and 9 within the anaerobic digester 7, since the anaerobic bacteria present within the anaerobic digester 7 require pH conditions that are substantially neutral or close to neutrality in order to be able to metabolize the sludge Vf.

For example, the pH of the sludge Vf might be decreased before the digestion step by means of placing the sludge Vf (or derivative thereof) in contact with the biogas Vg in the purification step.

Preferably, the derivative of the sludge Vf comprises the concentrated sludge Vfc, preferably it is constituted thereby.

Advantageously, during the digestion step, at least part of the carbon dioxide produced in the anaerobic digester 7 and present in the biogas Vg reacts with the sludge Vf or the derivative thereof and lowers the pH thereof.

Advantageously, the step of providing biogas Vg coincides with the digestion step. Therefore, the method provides for treating, in the purification step, the same biogas Vg produced in the digestion step. Advantageously, the method comprises a step of combustion (not illustrated in figure 1), in which the purified biogas Vg is burned, e.g. in a cogenerator 9, in order to generate at least electrical energy and a combustion gas Vgc comprising at least one fraction of carbon dioxide and one fraction of water vapor. In accordance with the preferred embodiment, the method also comprises a step of neutralization of the clarified waters Vc, and preferably also of the fraction of clarified waters Vex used in the purification step, in which an acid reagent Rac is added into the aforesaid clarified waters Vc in order to lower the pH thereof and obtain neutral clarified waters Vcn. Preferably, the acid reagent Rac is carbon dioxide. Still more preferably, the acid reagent Rac is the fraction of carbon dioxide present in the combustion gas Vgc, which in the neutralization step is placed in contact with the clarified waters Vc.

Advantageously, the method comprises a further treatment step of the neutral clarified waters Vcn in order to ensure the safe removal thereof. Such treatment step can provide for the use of treatment systems of known type and therefore is not described in detail.

The invention thus conceived therefore attains the pre-established objects.

## Claims

1. Method for treating biogas, **characterized in that** it comprises the following operating steps:
- providing:
- a biogas (Vg), said biogas (Vg) comprising at least one methane fraction (V_{CH4}) and at least one contaminant compound (C);
- waste water (Vr) to be treated, said waste water (Vr) comprising at least one organic pollutant compound (I); said organic pollutant compound (I) comprising at least one from among: a carbohydrate, a protein, a lipid, a surfactant, a dye, a biological material;
- adding an alkaline reagent (Ra) in said waste water (Vr) in order to obtain alkaline waste water (Va), said at least one organic pollutant compound (I) being transformed into modified molecules (M) due to the action of said alkaline reagent (Ra);
- coagulating said modified molecules (M) in order to obtain mature waste water (Vm) containing at least one flocculation (F) of said modified molecules (M);
- purifying said biogas (Vg), in which said biogas (Vg) is placed in contact with at least one fraction of said mature waste water (Vm) or one derivative thereof in order to transfer said at least one contaminant compound (C) in said at least one fraction of mature waste water (Vmx) or derivative;
- a step of separating said mature waste water (Vm) at least into clarified waters (Vc) and sludge (Vf), said flocculation (F) being mainly contained in said sludge (Vf);
- a step of digesting said sludge (Vf) or one derivative thereof by means of an anaerobic digester (7) in order to produce biogas (Vg) and a digestate (D).

2. Method for treating biogas according to claim 1, **characterized in that** the derivative of said mature waste water (Vm) comprises said clarified waters (Vc);
said biogas (Vg) being placed in contact with at least one fraction of said clarified waters (Vex) in said purification step.

3. Method for treating biogas according to claim 1 or 2, **characterized in that** the derivative of said mature waste water (Vm) comprises said sludge (Vf);
said biogas (Vg) being placed in contact with at least one fraction of said sludge (Vfx) in said purification step.

4. Method for treating biogas according to any one of the preceding claims, **characterized in that** said alkaline reagent (Ra) is a strong base selected from among sodium hydroxide, potassium hydroxide, lithium hydroxide and their mixtures or compounds.

5. Method for treating biogas according to any one of the preceding claims, **characterized in that** it comprises a step of dehumidifying of said biogas (Vg), following said purification step.

6. Plant for treating biogas **characterized in that** it comprises:
- a first flow piping (100) arranged for receiving and conveying waste water (Vr) to be treated, said waste water (Vr) comprising at least one organic pollutant compound (I);
- metering means, configured for metering an alkaline reagent (Ra) in said waste water (Vr) coming from said first flow piping (100) and obtaining alkaline waste water (Va);
- a maturation unit (2), in fluid communication with said first flow piping (100), and in which alkaline waste water (Va) coming from said first flow piping (100) is susceptible of being introduced in order to obtain mature waste water (Vm);
- a second flow piping (200), arranged for receiving and conveying biogas (Vg) to be treated, said biogas (Vg) comprising at least methane V_{CH4} and a contaminant compound (C);
- a purification unit (6), in fluid communication with said second flow piping (200) and at least said maturation unit (2), said purification unit (6) being arranged for receiving said biogas (Vg) and at least one fraction of said mature waste water (Vmx) or one derivative thereof and for placing them in contact with each other in order to transfer said contaminant compound (C) from said biogas (Vg) to said at least one fraction of mature waste water (Vmx) or derivative;
- a separation unit (3) in fluid communication with said maturation unit (2), said separation unit (3) being arranged for separating mature waste water (Vm) coming from said maturation unit (2) into at least clarified waters (Vc) and sludge (Vf);
- at least one anaerobic digestor (7) placed downstream of said separation unit (3) and arranged for treating said sludge (Vf) or derivatives thereof coming from said separation unit (3) in order to generate biogas (Vg) and a digestate (D).

7. Plant for treating biogas according to claim 6 or 7, **characterized in that** said purification unit (6) is in fluid communication with said separation unit (3) in order to receive at least one fraction of said clarified waters (Vex).

8. Plant for treating biogas according to claim 6 or 7, **characterized in that** said biogas (Vg) comprises a methane fraction (V_{CH4}) and at least one contaminant compound (C);
and that said second flow piping (200) is placed to connect between said anaerobic digestor (7) and said purification unit (6) in order to bring said biogas (Vg) to said purification unit (6).

9. Plant for treating biogas according to any one of the claims from 6 to 8, **characterized in that** said purification unit (6) comprises:
- at least one tank, which is arranged for containing said fraction of mature waste water (Vmx) or derivative,
- at least one bubbling dispenser placed within said tank and in fluid communication with said second flow piping (200) in order to convey said biogas (Vg) into said tank by means of bubbling.

10. Plant for treating biogas according to any one of the claims from 6 to 8, **characterized in that** said purification unit (6) comprises at least one washing column (60), which comprises:
- a treatment tank,
- a spraying head, placed within said treatment tank and placed in fluid connection with said separation unit (3) in order to nebulize, within said treatment tank, said fraction of mature waste water (Vmx) or derivative;
- an introduction mouth, placed within said treatment tank below said spraying head and placed in fluid connection with said second flow piping (200) in order to introduce said biogas (Vg) into said treatment tank;
- a plurality of packing bodies placed within said treatment tank between the introduction mouth and the spraying head.
